# EUROPEAN PATENT APPLICATION

(11) **EP 2 957 318 A1**
(43) Date of publication of application: **23.12.2015**
(21) Application number: 14741495.7
(22) Date of filing: 28.01.2014
(51) Int. Cl.: A61N 1/06, A61N 1/18

(54) **BATTERY-EMBEDDED PORTABLE HIGH-FREQUENCY THERAPEUTIC APPARATUS**

(30) Priority: 13.02.2013 KR 20130015428; 09.05.2013 KR 20130052545; 09.05.2013 KR 20130052546; 09.05.2013 KR 20130052547; 09.05.2013 KR 20130052544
(71) Applicant: HABALAN Med & Beauty Co., Ltd., Geumcheon-gu, Seoul 153-704 (KR)
(72) Inventor: KIM, Sang Du, Seoul 153-704 (KR)
(74) Representative: Betten & Resch
(86) International application number: PCT/KR2014/000768
(87) International publication number: WO 2014/126348

(57) **Abstract**

Disclosed herein is a portable high-frequency therapeutic apparatus having an internal battery. The therapeutic apparatus includes an internal battery, a drive module, a conductor and a housing. The internal battery includes a charging unit which charges the battery using external power, and a high-frequency power supply unit which generates high-frequency power using power of the battery. The conductor is connected to the high-frequency power supply unit and applies the high-frequency power to the human body. The housing contains the battery and the drive module therein. The conductor is mounted in a front end of the housing in such a way that the conductor is exposed out of the housing.

## Description

### Technical Field

The present invention relates generally to high-frequency therapeutic apparatuses which apply a high frequency to the human body to generate deep heat and treats an affected part using the deep heat and, more particularly, to a high-frequency therapeutic apparatus having an internal battery in which a battery for use in supplying power to generate a high frequency is installed in a housing so that the therapeutic apparatus is portable and thus can be used regardless of the place and time; and the battery and a drive module for generating high-frequency power are separably connected to each other by a connector so that the battery can be easily separated and replaced with a new one; and which can optionally adjust the magnitude of the high-frequency power applied to the skin of the human body depending on the characteristics or physical constitution of a patient or conditions of the affected part.

### Background Art

Generally, high-frequency therapeutic apparatuses are medical apparatuses which are used for obesity treatment, muscle strengthening, skin care, hair growth promotion, pain relief, etc. in such a way that high-frequency power is applied to the body of a patient and converted into bio-energy, and deep heat generated from the bio energy promotes fat metabolism and muscular motion.

If high-frequency power is applied to the human body, every time the direction of current is changed molecules of a corresponding bodily tissue vibrate and friction is created between the molecules. Consequently, heat is generated in the corresponding tissues of the body. This heat is referred to as deep heat, and the operation of generating such heat is referred to as deep heat generation.

The current of high-frequency power, unlike other types of current, can heat a specific portion of the desired bodily tissue without stimulating sensory nerves or a motor nerves causing contracting of muscles.

High-frequency energy that is converted into bio-heat energy increases the temperature of the corresponding bodily tissue, thus promoting the function of cells. Furthermore, the high-frequency energy expands the arteries and capillaries, thus increasing blood flow rate, thereby promoting circulation of blood and lymph fluid, and improving metabolism.

Various conventional techniques related to such high-frequency therapeutic apparatuses have been introduced, for example, in Korean Patent Registration No. 1068761, entitled "Electrode unit for high-frequency therapy", and Korean Patent Unexamined Publication No. 2009-0063521, entitled "Therapeutic apparatus using high-frequency deep heat."

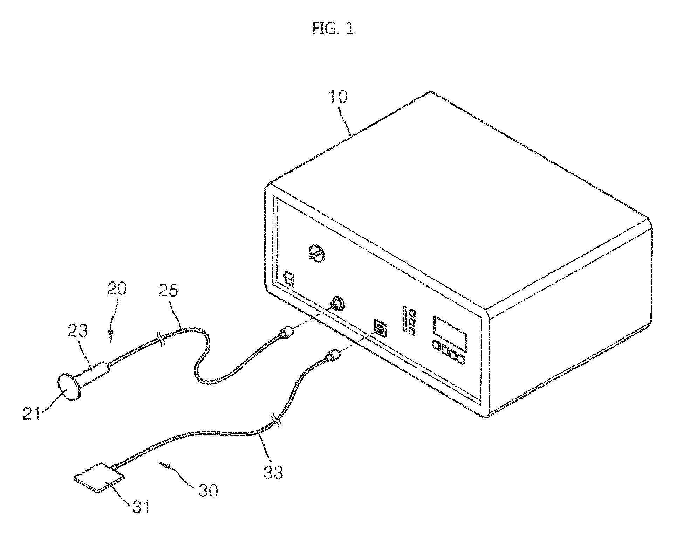

As shown in FIG. 1, generally, the high-frequency therapeutic apparatuses according to the conventional techniques including the above-mentioned techniques of Nos. 1068761 and 2009-0063521 include: a main body 10 which generates high-frequency power and outputs it; a high-frequency application unit 20 which has an application conductor 21, which is connected to the main body 10 by a cable 26 to apply high-frequency power to the human body, and is provided with a handle 23 which is gripped by a user; and a current carrying unit 30 which has a plate-shaped current carrying conductor 31 which is connected to the main body 10 by a cable 33 and provided to allow the flow of applied high-frequency power.

In such a conventional high-frequency therapeutic apparatus, the high-frequency application unit 20 which is gripped and handled by the user is separately provided from the main body 10 which generates the high-frequency power. In addition, the volume of the main body 10 is comparatively large. Thus, the conventional high-frequency therapeutic apparatus can be used only in a place where the apparatus is installed, and it is difficult to carry the apparatus and use it regardless of time and place.

Furthermore, the conventional high-frequency therapeutic apparatus is disadvantageous in that the current carrying conductor 31 of the current carrying unit 30 which is separately provided from the application conductor 21 of the high-frequency application unit 20 must be brought into contact with the skin of a patient before the application conductor 21 is brought into contact with the skin of the patient.

In other words, if a user could bring the application conductor 21 into contact with the skin of a patient without giving attention to the current carrying conductor 31, this would be very convenient. The conventional high-frequency therapeutic apparatus is however configured in such a way that the current carrying conductor 31 and the application conductor 21 are separately provided, thus forcing the user to give attention to both the current carrying conductor 31 as well as the application conductor 21 when desiring to use the apparatus.

Meanwhile, as high-frequency therapeutic apparatuses apply high-frequency power to the human body to generate deep heat and treat an affected part using the deep heat, there is a need for varying the magnitude of the high-frequency power applied to the human body depending on the characteristics or physical constitution of a patient or conditions of the affected part.

If there is the possibility of an electric shock or a burn, controlling high-frequency power applied to the human body is also needed.

However, the conventional high-frequency therapeutic apparatus interrupts application of high-frequency power only in an emergency situation rather than adjusting the magnitude of high-frequency power applied to the human body.

In other words, to prevent danger of an electric shock when high-frequency power is applied to the human body and to prevent the human body from being burned by deep heat generated by the application of high-frequency power, the conventional high-frequency therapeutic apparatus interrupts high-frequency power in such an emergency so that high-frequency power cannot be applied to the human body. The conventional high-frequency therapeutic apparatus however does not have any means for adjusting the magnitude of high-frequency power depending on the characteristics or physical constitution of a patient or conditions of the affected part.

Furthermore, in the conventional technique, a cylindrical solid rod is used as the application conductor which comes into contact with the skin of the human body and applies a high-frequency thereto. Given the fact that most high-frequency power is applied to the human body through the surface of the conductor, the conventional technique using the cylindrical solid conductor has problems of material waste and increases in production cost and weight.

In addition, the conductor of the conventional technique is configured such that it is installed by means of a bolt. Therefore, a conductor installation process is inconvenient, and there is the possibility of infiltration of water or a medical agent into the conductor because it has low water resistance.

### Disclosure

### Technical Problem

Accordingly, the present invention has been made keeping in mind the above problems occurring in the prior art, and an object of the present invention is to provide a portable high-frequency therapeutic apparatus having an internal battery in which a battery and a drive module for use in generating high-frequency power and charging the battery are housed in a housing which is gripped by a user, so that the apparatus can be reduced in size whereby the user can carry the apparatus and use it regardless of the time and place, and in which the battery and the drive module are separably connected to each other by a connector so that when the battery has malfunctioned or expired, the battery can be replaced with a new one through a simple process.

Another object of the present invention is to provide a portable high-frequency therapeutic apparatus having an internal battery in which an application conductor and a current carrying conductor are disposed in a front end of the housing so that they together can make contact with the skin of the human body, thus eliminating the need for having to bring the current carrying conductor into contact with the skin through a separate process.

A further object of the present invention is to provide a portable high-frequency therapeutic apparatus having an internal battery which is configured such that the user can optionally adjust the magnitude of high-frequency power applied to the human body, whereby an appropriate magnitude of high-frequency power can be applied to the skin of a patient depending on the characteristics or physical constitution of a patient or conditions of the affected part.

Yet another object of the present invention is to provide a portable high-frequency therapeutic apparatus having an internal battery in which the conductors can have hollow structures without affecting the performance to apply high-frequency power to the skin of the human body, so that the weight of the apparatus can be reduced and the material cost for the conductors and the production cost of the apparatus can be reduced; in which a support block is installed in each conductor to reinforce the strength reduced due to the hollow structure so that the conductors can be prevented from being damaged by external shock; and in which the conductors can be easily assembled with the housing, and a waterproof pad is provided between the housing and the conductors to prevent water or a medical agent from entering the housing through a gap between the housing and the conductors, wherein the waterproof pad is pressed by the support block so that the waterproofness of the apparatus can be markedly enhanced.

### Technical Solution

In order to accomplish the above objects, the present invention provides a portable high-frequency therapeutic apparatus, including: an internal battery; a drive module comprising a charging unit for charging the battery using an external power, and a high-frequency power supply unit for generating high-frequency power using power of the battery; a conductor connected to the high-frequency power supply unit, the conductor applying the high-frequency power to a human body; and a housing containing the battery and the drive module therein, with the conductor mounted in a front end of the housing in such a way that the conductor is exposed out of the housing.

The drive module may include: a boosting unit for boosting voltage of power discharged from the battery; a constant-voltage unit converting power of the boosting unit into a constant voltage and supplying the constant voltage to the high-frequency power supply unit; and a magnitude control unit adjusting a magnitude of boosted voltage output from the boosting unit, thus controlling a magnitude of high-frequency power output from the high-frequency power supply unit.

The battery may be connected to the drive module by a connector so that the battery can be separated from the drive module and replaced with a new one.

The conductor may include: a convex part protruding outwards from the housing through a through hole of the housing; a concave part formed in an inner surface of the convex part; and an extension part extending outwards from a peripheral edge of the convex part, the extension part being stopped by an inner surface of the front end of the housing. The portable high-frequency therapeutic apparatus may further include: a support block having a support part disposed in the concave part of the conductor, the support block supporting the conductor; and a waterproof pad disposed in the convex part of the conductor, the waterproof pad pressed between the extension part and the inner surface of the front end of the housing, thus filling a gap between the conductor and the housing.

### Advantageous Effects

As described above, a portable high-frequency therapeutic apparatus having an internal battery according to the present invention can be configured to be small and light. Furthermore, the therapeutic apparatus includes a drive module which has a battery and a charging unit for charging the battery. Therefore, the therapeutic apparatus can be used anywhere and at anytime with high portability. The battery and the drive module are connected to each other by a connector which can be easily separated therefrom. Thus, when the battery has expired or has malfunctioned, it can be easily replaced with a new one. In addition, a user can optionally adjust the magnitude of high-frequency power depending on the characteristics or physical constitution of a patient or conditions of the affected part so that an appropriate magnitude of high-frequency power can be applied to the skin of the patient, whereby satisfactory and reliable high-frequency therapy effects can be obtained. Moreover, the elements of the therapeutic apparatus can be easily assembled with each other, so that labor costs can be reduced and productivity can be enhanced. Thanks to superior durability, the therapeutic apparatus can be used for a long period of time. Consequently, the present invention is regarded as being very useful for development of the related industry.

### Description of Drawings

FIG. 1 is a high-frequency therapeutic apparatus according to the conventional invention;

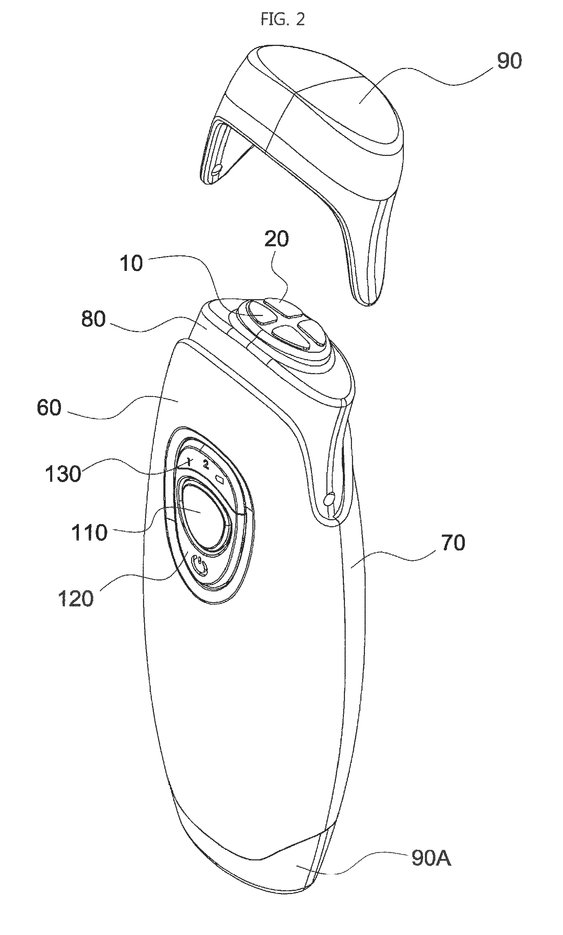

FIG. 2 is a perspective view illustrating a portable high-frequency therapeutic apparatus according to the present invention;

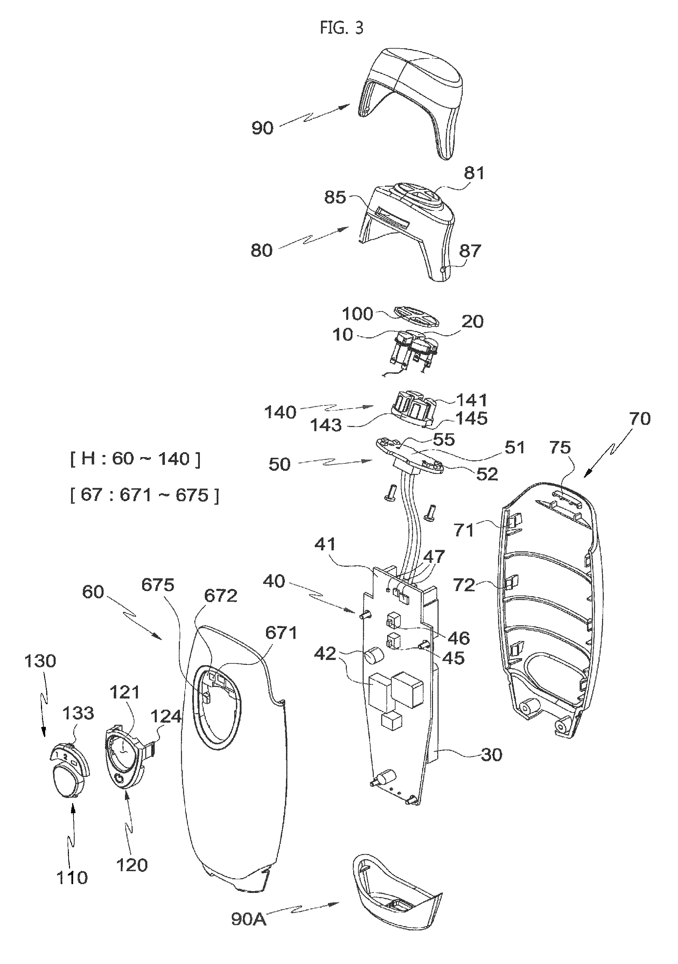

FIG. 3 is an explored perspective view showing the apparatus of FIG. 2;

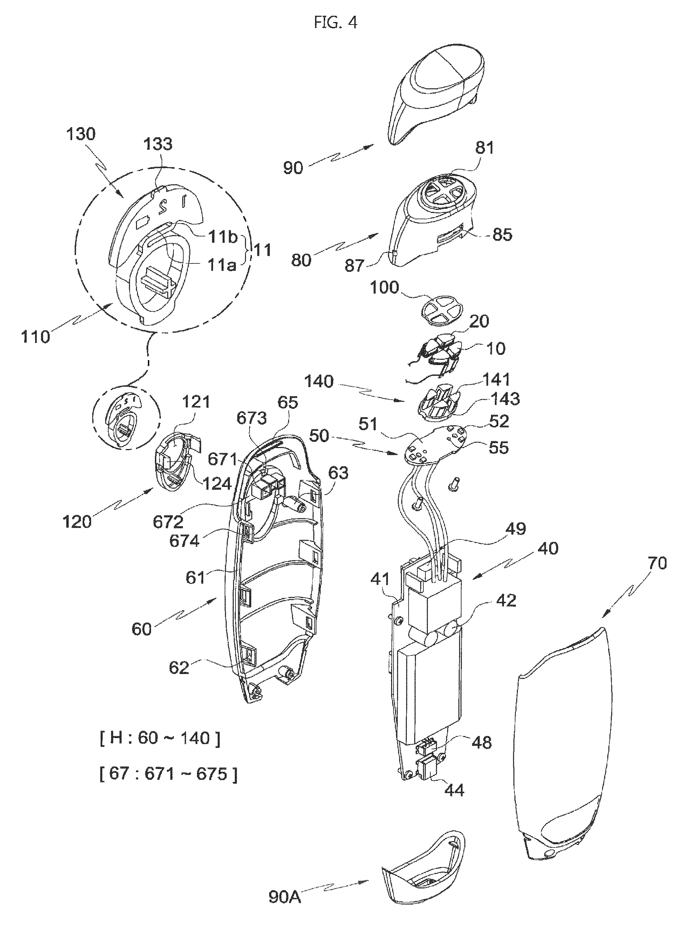

FIG. 4 is an explored perspective view showing the apparatus of FIG. 2 from a different direction;
FIG. 5 is a sectional view of FIG. 2;

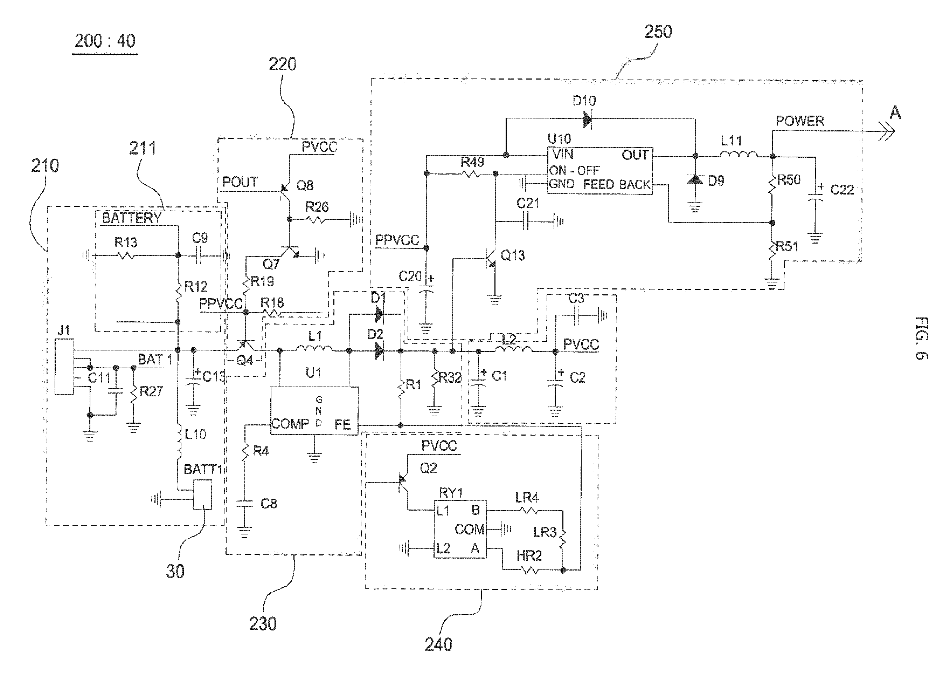

FIG. 6 is a circuit diagram of an input side of a drive module;

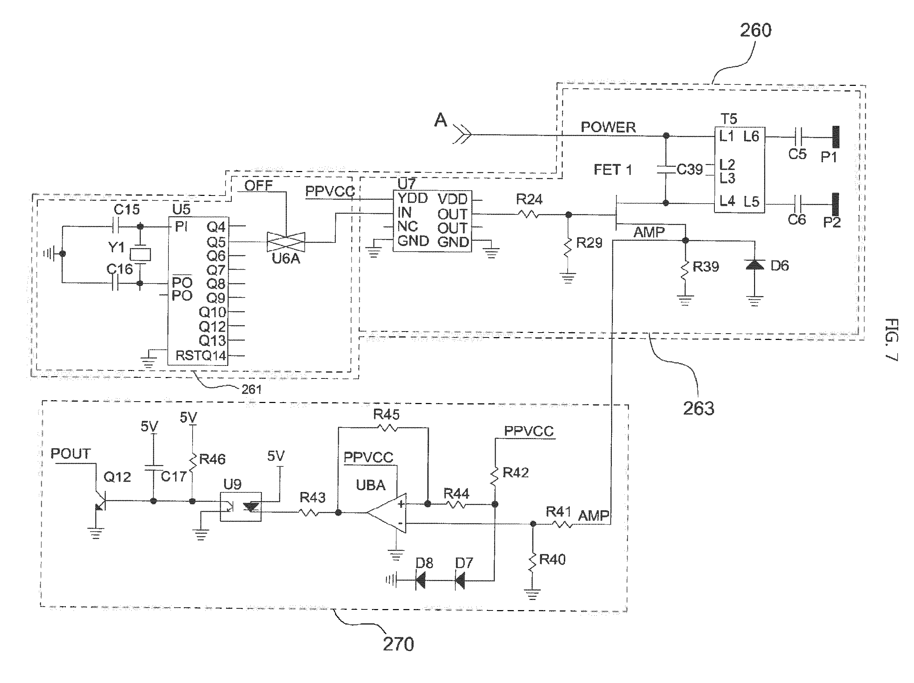

FIG. 7 is a circuit diagram of an output side of the drive module which is connected to the circuit diagram of FIG. 6; and

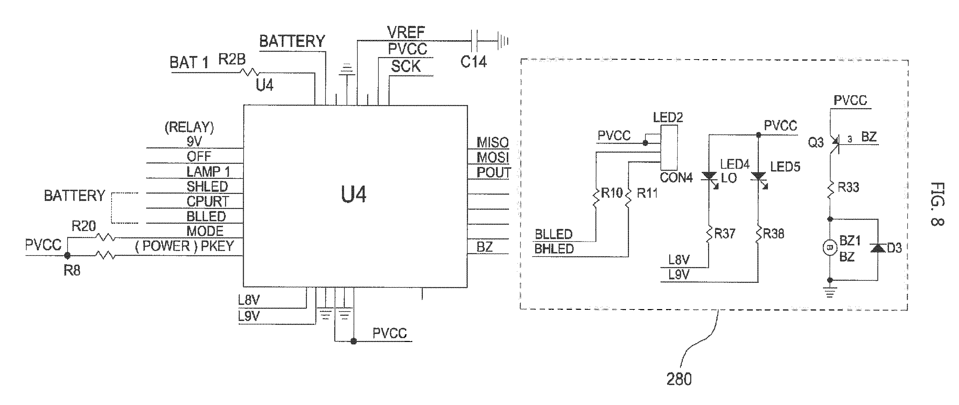

FIG. 8 illustrates circuit diagrams of a CPU and an indication unit of the drive module.

### <Description of the Reference Numerals in the Drawings>

10: application conductor 20: current carrying conductor
30: battery 40: high-frequency power module
50: stimulation module 60: upper casing
70: lower casing 80: head
90: cap 110: control button
120: power button 130: indication display
H: housing

### Best Mode

Hereinafter, a portable high-frequency therapeutic apparatus having an internal battery according to the present invention will be described in detail with reference to the attached drawings.

The present invention will now be described in detail based on aspects (or embodiments). The present invention may, however, be embodied in many different forms and should not be construed as being limited to only the embodiments set forth herein, but should be construed as covering modifications, equivalents or alternatives falling within ideas and technical scopes of the present invention.

The same reference numerals throughout the drawings, that is, the same reference numerals for the second digit or the first digit, or for the second digit, the first digit and a letter of the alphabet, denote elements having the same function. If not specifically mentioned otherwise, the elements denoted by the reference numerals are to be assumed to comply with the above-mentioned reference scheme.

In the drawings, the thicknesses of lines or the sizes of elements may be exaggerated or simplified to more clearly and conveniently illustrate the present invention, but the bounds of the present invention must not be interpreted as being limited thereto.

The terminology used herein is for the purpose of describing particular aspects (or embodiments) only and is not intended to be limiting of the present invention. As used herein, the singular forms are intended to include the plural forms as well, unless the context clearly indicates otherwise. It will be further understood that the terms "comprises," "comprising,", "includes" and/or "including," when used herein, specify the presence of stated features, integers, steps, operations, elements, and/or components, but do not preclude the presence or addition of one or more other features, integers, steps, operations, elements, components, and/or groups thereof.

Unless otherwise defined, all terms including technical and scientific terms used herein have the same meaning as commonly understood by one of ordinary skill in the art to which the present invention belongs. It will be further understood that terms used herein should be interpreted as having a meaning that is consistent with their meaning in the context of this specification and the relevant art and will not be interpreted in an idealized or overly formal sense unless expressly so defined herein.

As shown in the drawings, the portable high-frequency therapeutic apparatus having an internal battery according to the present invention includes a battery 30, a drive module 40, conductors 10 and 20, and a housing (H; 60 through 140).

The battery 30 provides electrical power for generating high-frequency power. A rechargeable secondary battery is used as the battery 30.

The drive module 40, using electrical power supplied from the battery 30, generates high-frequency power and outputs the power to the application conductor 10 and the current carrying conductor 20. The drive module 40 uses external electrical power input from the outside and charges the battery 30. The drive module 40 discharges the battery 30 or controls the magnitude of the high-frequency power in response to a signal generated by manipulating a power button 120 and a control button 110.

The drive module 40 has a mechanical structure and an electrical structure. The mechanical structure includes a PCB 41 and different kinds of electronic devices 42 which are mounted on a PCB 41. The electrical structure includes a charging unit, a high-frequency power supply unit, etc. each of which is formed by connecting at least some of the electronic devices 42 to each other. The electrical structure of the drive module 40 will be described later herein.

A charge port 44 is provided in a rear end of the PCB 41 of the drive module 40 so that a charging cable connected to an external power supply to charge the battery 30 is connected to the charge port 44. A power switch 45 which turns the power on or off, a control switch 46 which controls the magnitude of high-frequency power, and an indication lamp 47 which indicates information about operating conditions are provided in an upper surface of a front portion of the PCB 41. A connector 48 which electrically connects the battery 30 to the drive module 40 so as to be easily separable is provided on a rear surface of the PCB 41.

Tact switches, which are pushed by external force and automatically returned to their original states when the external force is removed, are used as the power switch 45 and the control switch 46. A small chip LED is used as the indication lamp 47.

A stimulation module 50 is connected to the front end of the drive module 40 by connectors 49 and 59 and a cable.

The stimulation module 50 is disposed in a head 80 of the housing. The stimulation module 50 includes a PCB 51 and stimulation lamps 52. The stimulation lamps 52 are mounted on the PCB 51 and disposed on opposite sides of the current carrying conductor 20 and the application conductor 10. The stimulation lamps 52 emit light when high-frequency power is applied to the skin of the human body. Small chip LEDs are used as the stimulation lamps 52. Light emitted from the stimulation lamps 52 stimulates and activates skin cells.

The conductors 10 and 20 apply high-frequency power to the human body. The conductors include the application conductor 10 and the current carrying conductor 20. As shown in FIG. 1, in the conventional technique, generally the application conductor is provided on a handle and is brought into contact with a desired portion of the skin. The current carrying conductor 20 is separately provided on a main body which generates high-frequency power, and is configured to be grabbed with the hand or connected to one of other body parts. Therefore, the application conductor and the current carrying conductor are separately connected to the skin of the human body, thus being inconvenient.

To solve the problem of the conventional technique, in the present invention, both the application conductor 10 and the current carrying conductor 20 are provided in the head 80 of the housing H so that the conductor 10 and 20 together can be brought into contact with the skin in a single process without forcing a user to individually bring the conductor 10 and 20 into contact with the skin through separate processes.

The application conductor 10 and the current carrying conductor 20 which are electrically connected to the drive module 40 come into contact with the skin, apply high-frequency power to the human body, and enable the applied high-frequency power to flow through the human body, thus stimulating the tissues of the human body between the application conductor 10 and the current carrying conductor 20, and generating deep heat.

The high-frequency energy that has been converted to the deep heat (that is, bio-heat energy) elevates the temperature of the body tissues, thus enhancing the function of cells. Furthermore, the high-frequency energy expands the arteries and capillaries, thus increasing blood flow rate, thereby stimulating the circulation of blood and lymph fluid, and improving metabolism.

Each of the conductors 10 and 20 includes a convex part 11 which protrudes to the outside through a through hole 81 of the head 80 of the housing and comes into contact with the skin of the human body, a concave part 12 which is formed inside the convex part 11 and has a depression, an extension part 13 which extends outwards from the edge of a rear end of the convex part 11 and is stopped by an inner surface of the head 80, and a connector 14 which is connected to the rear end of the convex part 11 and electrically connected to the drive module 40.

Each of the application conductor 10 and the current carrying conductor 20 comprises two conductors. The two application conductors 10 are electrically connected to each other so that high-frequency power is applied to the two application conductors 10 at the same time. The two current carrying conductors 20 are also electrically connected to each other.

The application conductor 10, the current carrying conductor 20 and the front portion of the head 80 of the housing H to which the application conductor 10 and the current carrying conductor 20 are mounted and configured in such a way that they are exposed to the outside to make contact with the skin of the patient. Therefore, if the skin is wet or a medical agent is applied to the skin, there is the possibility of water or the medical agent entering the housing H through a gap between the head 80 of the housing H and the application conductor 10 or the current carrying conductor 20. That is, water or a medical agent may enter the housing through the through hole 81 which is formed in the head 80.

To avoid the above problems, the present invention further includes a waterproof pad 100 which prevents water or a medical agent from entering the housing H, and a support block 140 which pressurizes the waterproof pad 100 to reliably seal the housing H.

In more detail, the waterproof pad 100 is fitted over the convex parts 11 of the application conductor 10 and the current carrying conductor 20 and interposed between the extension parts 13 and the inner surface of the head 80. The support block 140 supports the application conductor 10 and the current carrying conductor 20 toward the front side of the conductors so that the waterproof pad 100 is pressurized between the extension parts 13 and the inner surface of the head 80, thus reliably closing the gap.

A rear end of the support block 140 is supported on the PCB 51 of the stimulation module 50. The PCB 51 of the stimulation module 50 is tightly coupled to the inner surface of the head 80 by a screw, thus pushing the support block 140 forwards so that the application conductor 10 and the current carrying conductor 20 can be pushed forwards.

The support block 140 pushes the application conductor 10 and the current carrying conductor 20 forwards so that the waterproof pad 100 closes the gap. The support block 140 also functions to prevent the application conductor 10 or the current carrying conductor 20 from being deformed or damaged when a shock is applied to the application conductor 10 or the current carrying conductor 20.

The support block 140 includes support parts 141, a contact part 143 and insert protrusion 145. The support parts 141 are disposed in the respective concave parts 12 of the application conductor 10 and the current carrying conductor 20. The support parts 141 supports the application conductor 10 and the current carrying conductor 20 to prevent the application conductor 10 and the current carrying conductor 20 from being damaged by external shocks. The contact part 143 is formed on the rear ends of the support parts 141 and is brought into contact with the PCB 51 of the stimulation module 50. The insert protrusion 145 is provided on the rear surface of the contact part 143 and inserted into an insert hole 55 formed in the PCB 51.

The drive module 40, the battery 30 and the stimulation module 50 are housed in the housing H.

The housing H includes an upper casing 60 and a lower casing 70 which are assembled with each other; the head 80 which is inserted into and coupled to upper ends of the assembled upper and lower casings 60 and 70; an upper cap 90 which is detachably fitted over an outer circumferential surface of the head 80; and a lower cap 90A which is assembled with lower ends of the assembled upper and lower casings 60 and 70 in a hook coupling fashion.

For engagement coupling between the upper and lower casings 60 and 70, stepped parts 61 and 71 which engage with each other are respectively formed in opposite ends of the inner surface of the upper and lower casings 60 and 70. A hook protrusion 72 and a hook depression 62 are respectively provided on opposite edges of the inner surfaces of the lower and upper casings 70 and 60 so that the upper and lower casings 60 and 70 can be easily assembled with each other in a hook coupling fashion.

A plurality of support bosses 63 which support an upper surface of the PCB 41 are provided on the perimeter of the inner surface of the upper casing 60.

The head 80 is coupled to the front ends of the upper and lower casings 60 and 70 which have been assembled with each other.

The through hole 81 into which the application conductor 10 and the current carrying conductor 20 are inserted is formed in the front surface of the head 80. Assembly slots 85 are respectively formed in the upper and lower surfaces of the head 80. Protrusions 65 and 75 are respectively provided on front ends of the inner surfaces of the upper and lower casings 60 and 70. The protrusions 65 and 75 are respectively inserted into the corresponding assembly slots 85 so that the head 80 is coupled to the upper and lower casings 60 and 70. Locking depressions 87 are formed on opposite side portions of the outer surface of the head 80 so that the upper cap 90 can be removably fitted over the head 80 by means of the locking depressions 87.

A mounting part is formed in the upper casing 60. The power button 120 and the control button 110 for use in controlling the therapeutic apparatus and an indication display 130 for use in displaying information about operating conditions of the therapeutic apparatus are provided in the mounting part.

The power button 120 pushes the power switch 45, which is provided in the drive module 40, so as to switch on or off the power of the therapeutic apparatus according to the present invention (in other words, discharge the battery 30 or interrupt the discharge thereof). The control button 110 pushes the control switch 46, thus controlling the magnitude of the high-frequency power. The indication display 130 indicates the operating conditions of the therapeutic apparatus according to the present invention (for example, whether the power is turned on or not, the magnitude of high-frequency power, charging conditions of the battery, etc.).

The mounting part 67 of the upper casing 60 includes a support plate 672 which has a transmission hole 671 through which light emitted from the indication lamp 47 can pass. The support plate 672 supports the indication display 130 on an upper surface thereof.

An insert hole 673 into which an insert protrusion 133 formed on the front surface of the indication display 130 is inserted is formed in the front surface of the support plate 672.

A hook-locking protrusion 674 to which a hook protrusion 124 provided on the power button 120 is hooked is provided on the rear surface of the support plate 672.

A support protrusion 675 which supports the power button 120 is provided behind the hook-locking protrusion portion 674.

A hollow hole 121 is formed in the power button 120. The control button 110 is disposed in the hollow hole 121.

The control button 110 is integrally coupled to the indication display 130 by an elastic rod 111.

Integrally formed with the control button 110 and the indication display 130, the elastic rod 111 is configured to have an appropriate length to prevent it from being easily broken and to maintain its elastic restoring force. Preferably, the elastic rod 11 includes a lateral part 111a which is disposed between the control button 110 and the indication display 130 and extends a predetermined length in the lateral direction of the apparatus, and longitudinal parts 111b which extend both ends of the lateral part 111a in the longitudinal direction of the apparatus and are respectively coupled to the control button 110 and the indication display 130.

The distance between the control button 110 and the indication display 130 is relatively short. Given this, if the elastic rod 111 is short and couples the control button 110 to the indication display 130 in a crow line, the displacement of the elastic rod 111 is excessively increased when the user pushes the control button 110. In this case, the elastic rod 11 may snap or lose its elastic restoring force.

A surface of the indication display 130 is partitioned into a masking area which blocks light emitted from the indication lamp 47 of the drive module 40 and a transmission area which allows light to pass through. Allowing transmission of light, the transmission area has a numeral or battery symbol. Thereby, while light emitted from the indication lamp 47 transmits through the transmission area, the transmission area informs the user of, for example, the magnitude of the high-frequency power, charging conditions of the battery, etc.

When the user pushes a rear portion of the power button 120, the rear portion of the power button 120 rotates on the support protrusion 675 and thus pushes the power switch 45. When the user releases the lower portion of the power button 120, the lower portion of the power button 120 is returned to its original state by the hook protrusion 124 which is hooked to the front end of the support protrusion 675. When the user pushes the control button 110, the control button 110 pushes the control switch 46 while overcoming the elasticity of the elastic rod 111. When the user releases the control button 110, the control button 110 is returned to its original state by the elasticity of the elastic rod 111.

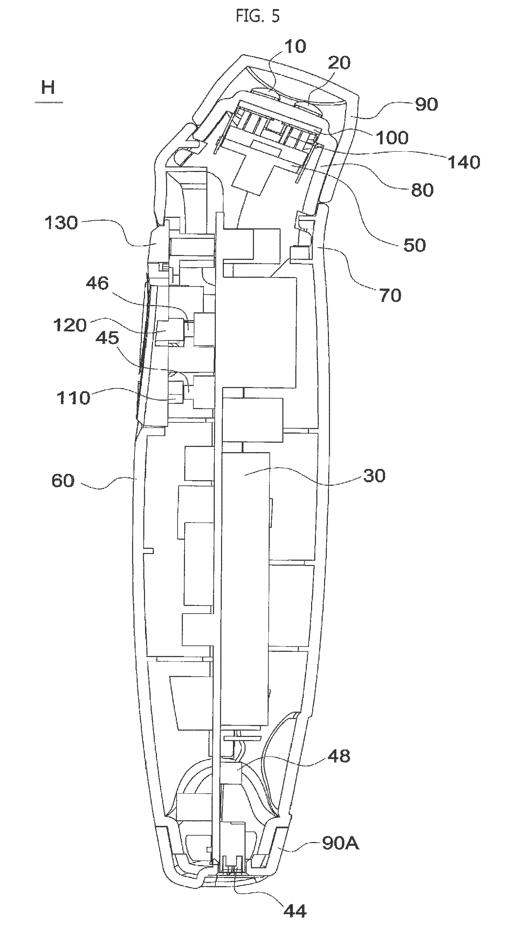

Hereinafter, the electrical structure, that is, a circuit diagram 200, of the drive module 40 according to the present invention will be described with reference to FIG. 5

The electrical structure of the drive module 40 includes a charging unit 210, a discharging unit 220, a boosting unit 230, a magnitude control unit 240, a constant-voltage unit 250, a high-frequency power supply unit 260, an overload detection unit 270, an indication unit 280 and a CPU U4.

The charging unit 210 charges the battery 30 with power input from the outside through the charging port 44.

The charging unit 210 includes a coil L10 which removes a noise from external DC power input through the charging port (J1;44) before the external DC power is supplied to the battery (B;30).

Recently, there is a growing trend for electronic devices to be charged by the power of a personal computer (PC). In this embodiment, in accordance with this trend, given the fact that the power of a PC is used as the external power supply, the charging unit 210 is formed merely of the coil L10. However, if commercial power is used as the external power supply, the charging unit 210 must not only include the coil L10 but also include a convertor which reduces the voltage of the commercial power and converts AC current thereof into DC current.

The charging unit 210 further includes a check part 211 which checks the voltage charged into the battery and transmits it to the CPU U4. Receiving information about the voltage of the battery from the check part 211, the CPU U4 instructs the indication lamp (LED 2;47) to be red, yellow or green depending on the magnitude of the voltage, thus indicating charging conditions of the battery.

The discharging unit 220 discharges the battery 30 when the power button 120 is pressed.

The discharging unit 220 is provided on an output line of the battery. The discharging unit 220 includes a discharge transistor Q4 which is turned on or off to discharge the battery or interrupt the discharge thereof, and control transistors Q7 and Q8 which are turned on or off to control the on/off status of the discharge transistor Q4 under control of the CPU U4 depending on whether the power button 120 is pushed.

The boosting unit 230 boosts the voltage of the battery discharged through the discharging unit 220 and supplies it to the constant-voltage unit 250.

The boosting unit 230 includes a boosting coil L1 which boosts power discharged from the battery, a boost control part U1 which controls the supply of current from the battery to the boosting coil L1 or interruption of the supply of current so as to induce electromotive force to be generated in the boosting coil L1 and thus boost voltage, and diodes D1 and D2 which rectify the voltage boosted by the boosting coil L1.

Voltage output from the boosting unit 230 is supplied to the constant-voltage unit 250 and a drive power unit 290.

The drive power unit 290, using capacitors C1, C2 and C3 and the coil L2, converts voltage output from the boosting unit 230 into DC drive power required to drive the CPU U4.

The magnitude control unit 240, when the user pushes the control button 110, adjusts the magnitude of power output from the boosting unit 230, thus controlling the magnitude of high-frequency power output from the high-frequency power supply unit 260.

The magnitude control unit 240 includes a transistor Q2 which is turned on or off depending on whether the control button 110 is pressed or released, and a relay RY1 which is switched on or off depending on whether the transistor Q2 is turned on or off and transmits a switching signal to the boost control part U1 of the boosting unit 230.

The boost control part U1 of the boosting unit 230 that receives a signal from the magnitude control unit 240 adjusts the induced electromotive force generated from the boosting coil L1, thus controlling the magnitude of voltage which is boosted.

The constant-voltage unit 250 converts voltage output from the boosting unit 230 into constant voltage and supplies the constant voltage to the high-frequency power supply unit 260.

The constant-voltage unit 250 includes a regulator U10 which regulates voltage input from the boosting unit 230 to convert it into constant voltage, and a transistor Q10, a coil L11, diodes D9 and D10, a resistor, a capacitor, etc. which form a peripheral circuit of the regulator U10.

The high-frequency power supply unit 260 switches and boosts constant voltage output from the constant-voltage unit 250 to generate high-frequency power, and then supplies the high-frequency power to the application conductor 10 and the current carrying conductor 20.

The high-frequency power supply unit 260 includes a transformer T5 which boosts constant voltage input from the constant-voltage unit 250, an oscillator 261 which produces an oscillating electronic signal so that the output power has a high frequency, and a driver 263 which switches the high-frequency power in accordance with the oscillation frequency produced from the oscillator 261 so that the voltage of the high-frequency power can be boosted by the transformer T5.

The overload detection unit 270 detects whether the high-frequency power supply unit 260 is overloaded or not and transmits a detection signal to the CPU U4, thus preventing the high-frequency power supply unit 260 from being overloaded.

The overload detection unit 270 includes a comparator UBA which compares voltage resulting from switching of a switching device FET1 of the driver 263 in the high-frequency power supply unit 260 with a reference voltage; a photocoupler U9 which transmits a signal to the CPU U4 when the result of comparison of the comparator UBA shows that voltage resulting from switching of the switching device FET1 of the driver 263 is greater than the reference voltage, in other words, the high-frequency power supply unit 260 is overloaded; and devices which form peripheral circuits of the comparator UBA and the photocoupler U9.

If the photocoupler U9 of the overload detection unit 270 transmits an overload signal to the CPU U4, the CPU U4 transmits a signal to the driver to reduce the switching speed such that the high-frequency power supply unit 260 is not overloaded.

The indication unit 280 includes indication lamps (LED2, LED4 and LED5; 47) which indicates information about operating conditions of the high-frequency therapeutic apparatus, and a buzzer BZ1 which sounds an alarm when there is a need for charging the battery attributable to an overdischarge of the battery, when the high-frequency power supply unit 260 is overloaded, or when other errors occur.

The indication lamp 47 of the indication unit 280 includes a charging condition indication LED LED2 which emits red, yellow or green light to indicate charging conditions of the battery, and two magnitude indication LEDs (LED4 and LED5) which indicate in two steps the magnitude of high-frequency power output from the high-frequency power supply unit 260.

The CPU U4 generally controls the drive module 40. For example, the CPU U4 receives a signal from the power switch 45 or the control switch 46 when the power button 120 or the control button 110 is pressed, and controls the discharging unit 220, the boosting unit 230, the constant-voltage unit 250, the high-frequency power supply unit 260, etc. in response to an input signal. When the overload detection unit 270 is overloaded, the CPU4 controls the high-frequency power supply unit 260 to recover it from the overloaded state and controls the indication unit 280 to indicate whether the high-frequency power supply unit 260 is in the overloaded state or not.

Although the preferred embodiment of the portable high-frequency therapeutic apparatus according to the present invention has been disclosed for illustrative purposes, those skilled in the art will appreciate that various modifications, additions and substitutions are possible, without departing from the scope and spirit of the invention as disclosed in the accompanying claims.

## Claims

1. A portable high-frequency therapeutic apparatus, comprising:
an internal battery;
a drive module comprising a charging unit for charging the battery using an external power, and a high-frequency power supply unit for generating high-frequency power using power of the battery;
a conductor connected to the high-frequency power supply unit, the conductor applying the high-frequency power to a human body; and
a housing containing the battery and the drive module therein, with the conductor mounted in a front end of the housing in such a way that the conductor is exposed out of the housing.

2. The portable high-frequency therapeutic apparatus as set forth in claim 1, wherein the drive module comprises:
a boosting unit for boosting voltage of power discharged from the battery;
a constant-voltage unit converting power of the boosting unit into a constant voltage and supplying the constant voltage to the high-frequency power supply unit; and
a magnitude control unit adjusting a magnitude of boosted voltage output from the boosting unit, thus controlling a magnitude of high-frequency power output from the high-frequency power supply unit.

3. The portable high-frequency therapeutic apparatus as set forth in claim 1 or 2, wherein the battery is connected to the drive module by a connector so that the battery can be separated from the drive module and replaced with a new one.

4. The portable high-frequency therapeutic apparatus as set forth in any one of claims 1 through 3, wherein the conductor comprises
a convex part protruding outwards from the housing through a through hole of the housing;
a concave part formed in an inner surface of the convex part; and
an extension part extending outwards from a peripheral edge of the convex part, the extension part being stopped by an inner surface of a head,
the portable high-frequency therapeutic apparatus further comprising:
a support block having a support part disposed in the concave part of the conductor, the support block supporting the conductor; and
a waterproof pad disposed in the convex part of the conductor, the waterproof pad pressed between the extension part and the inner surface of head, thus filling a gap between the conductor and the housing.
